# EUROPÄISCHE PATENTSCHRIFT

(11) **EP 3 720 387 B1**
(45) Veröffentlichungstag und Bekanntmachung des Hinweises auf die Patenterteilung: **12.01.2022**
(21) Anmeldenummer: 18808381.0
(22) Anmeldetag: 04.12.2018
(51) Int. Cl.: A61F 2/12, A61N 5/06

(54) **IMPLANTAT MIT RESERVOIR**
IMPLANT WITH RESERVOIR
IMPLANT DOTÉ D'UN RÉSERVOIR

(30) Priorität: 05.12.2017 EP 17205348
(43) Veröffentlichungstag der Anmeldung: 14.10.2020
(73) Patentinhaber: Repuls Lichtmedizintechnik GmbH, 1230 Wien (AT)
(72) Erfinder: RUMPOLD, Brigitte, 1230 Wien (AT)
(74) Vertreter: SONN Patentanwälte OG
(86) Internationale Anmeldenummer: PCT/EP2018/083418
(87) Internationale Veröffentlichungsnummer: WO 2019/110550

(56) Entgegenhaltungen:
- DE-A1-102005 008 955
- US-A1- 2008 241 212
- US-A1- 2011 160 854

## Beschreibung

Die Erfindung betrifft ein biokompatibles Implantat für einen Patienten, wobei das Implantat zumindest ein von einer Umhüllung definiertes Reservoir, das eine pharmazeutisch annehmbare Lösung enthält, aufweist.

Es ist bekannt, dass es nach der Implantation von Brustimplantaten wie auch anderen Implantaten zu schwerwiegenden Komplikationen aufgrund von Infektionen kommen kann. Oft können solche Infektionen allein mit systemischer Gabe von Antibiotika nur unzureichend bekämpft werden, so dass in vielen Fällen letztlich die Implantate sogar wieder operativ entfernt werden müssen. Ein Überblick über diese Problematik findet sich beispielsweise in Pittet et al., "Infection in breast implants." The Lancet Infectious Diseases 5.2 (2005): 94-106.

Die US 2011/016854 A1 offenbart ein Implantat der eingangs erwähnten Art, welches das Risiko solcher Infektionen verringern soll. Insbesondere wird darin ein Brustimplantat offenbart, das ein Reservoir aufweist, welches eine pharmazeutisch annehmbare Lösung mit einem Antibiotikum langsam in den Körper der Patientin freigibt. Dieses Reservoir ist mittels eines Schlauchs befüllbar. Nach der Implantation des Brustimplantats bildet der Schlauch einen künstlichen Eingang zum Brustimplantat im Körper der Patientin, über den die pharmazeutisch annehmbare Lösung in das Reservoir geleitet werden kann, um dann über die Zeit aus dem Reservoir freigegeben werden. Nach einer gewissen Zeit kann der Schlauch entfernt werden, so dass sich die Operationswunde der Patientin völlig schließen kann.

Das im vorgenannten Dokument offenbarte Implantat weist jedoch gravierende Nachteile auf: Zum einen stellt der aus dem Körper ragende Schlauch bzw. die noch nicht verschlossene Wunde selbst ein Infektionsrisiko dar. Zudem können mit dem Implantat assoziierte Infektionen noch Monate oder sogar Jahre später auftreten, also nach einer Zeitdauer, die länger ist als die Dauer, für die man den Schlauch am Implantat belassen könnte. Und zum anderen ist die Therapie mit Antibiotika allein (selbst, wenn sie direkt am Ort der Infektion freigesetzt werden wie im Fall des im vorgenannten Dokument offenbarten Implantats) insbesondere gegen multi-resistente Keime oft nicht ausreichend effektiv.

Es ist demgemäß Aufgabe der vorliegenden Erfindung, das durch Implantate bzw. deren Implantation verursachte Infektionsrisiko (bzw. das Risiko, dass eine etwaige Infektion gravierende Folgen nach sich zieht) gegenüber dem Stand der Technik weiter zu reduzieren bzw. die Behandlung von Implantat-assoziierten Infektionen zu verbessern.

Daher stellt die vorliegende Erfindung ein biokompatibles Implantat für einen Patienten zur Verfügung, wobei das Implantat zumindest ein von einer Umhüllung definiertes Reservoir, das eine pharmazeutisch annehmbare (üblicherweise wässrige) Lösung mit zumindest einem Photosensibilisator enthält, aufweist. Des Weiteren ist zumindest ein Teil der Umhüllung des zumindest einen Reservoirs durch eine Membran gebildet. Diese Membran ist von einer Nadel punktierbar, so dass durch Punktion mit der Nadel zumindest ein Teil der Lösung aus dem Reservoir in die Umgebung des Implantats freisetzbar ist. Dadurch wird die genannte Aufgabe gelöst.

Das erfindungsgemäße Implantat ermöglicht die zielgerichtete minimalinvasive lokale Behandlung von Implantat-assoziierten Infektionen mittels photodynamischer Therapie (ein allgemeiner Überblick über die Behandlung von Infektionen mittels photodynamischer Therapie findet sich beispielsweise in Dai et al. "Photodynamic therapy for localized infections-state of the art." Photodiagnosis and Photodynamic Therapy 6.3 (2009): 170-188.). Dabei wird üblicherweise wie folgt vorgegangen: Das erfindungsgemäße Implantat wird dem Patienten nach im Stand der Technik bekannten chirurgischen Verfahren eingesetzt und erfüllt seine übliche Funktion (z.B. Rekonstruktion der weiblichen Brust, wenn es ein Brustimplantat ist). Wenn sich nach der Operation beim Patienten Symptome einer Infektion zeigen, führt der behandelnde Arzt eine Nadel in den Körper des Patienten ein, um die zumindest einen Teil der Umhüllung des Reservoirs bildende Membran des erfindungsgemäßen Implantats zu punktieren. Aus dem durch die Punktion mit der Nadel erzeugten Loch in der Membran sickert nun die Lösung mit dem Photosensibilisator und verteilt sich im umliegenden, infizierten Gewebe in der Umgebung des Impantats. Nach einer gewissen Zeit (z.B. 1-24 Stunden) wird der Körper des Patienten von außen mit einer Lichtquelle, die Licht mit der Anregungswellenlänge des Photosensibilisators emittiert, bestrahlt. Das Licht dringt durch die Haut des Patienten ins Gewebe ein (die Eindringtiefe ist u.a. abhängig von der Lichtstärke und der Wellenlänge des Lichts). Wenn das Licht mit dem umliegenden, infizierten Gewebe in der Umgebung des Impantats in Kontakt kommt, wird der Photosensibilator angeregt und es entstehen in Folge u.a. reaktive Sauerstoffspezies. Dadurch werden die Mikroorganismen, die das Gewebe infiziert haben, geschädigt und die Infektion wird somit behandelt. Eine zur Bestrahlung des infizierten Gewebes geeignete Vorrichtung, wenn als Photosensibilisator z.B. Methylenblau eingesetzt wird, ist beispielsweise in der WO 2008/151340 A1 offenbart.

Die Membran des erfindungsgemäßen Implantats muss derart beschaffen sein, dass sie von einer Nadel (z.B. von einer gewöhnlichen Punktionsnadel, die üblicherweise von einem Arzt geführt wird) punktiert werden kann. Die Membran muss jedoch hinreichend stark sein, so dass möglichst vermieden wird, dass sie z.B. bei der Implantation reißt. Beispielsweise ist die Membran derart beschaffen, dass eine Kraft zwischen 0,5 N und 2 N mit einer im rechten Winkel auftretenden 25-Gauge-Nadel aufzuwenden ist, um die Membran zu punktieren. Typischerweise ist die Membran elastisch, sie kann beispielsweise ein Film aus einem biokompatiblen Kunststoff sein.

Gemäß einer bevorzugten Ausführungsform des erfindungsgemäßen Implantats bildet ein erster Teil der Umhüllung, welcher die Membran aufweist, eine Außenwand des Implantats und ein zweiter Teil der Umhüllung eine Innenwand des Implantats, wobei die Innenwand zumindest teilweise eine Implantatfüllung umgibt. Dadurch wird der Materialverbrauch für die Erzeugung des Implantats verringert. Nach der Implantation steht die Außenwand typischerweise direkt mit der Umgebung (d.h. dem umliegenden Gewebe) in Berührung, während dies für die Innenwand nicht zutrifft. Diese Innenwand kann mit der Implantatfüllung direkt in Berührung stehen. Die Implantatfüllung wiederum kann fest, flüssig oder gelförmig sein.

Nach einer weiteren bevorzugten Ausführungsform weist das Implantat eine Vielzahl an Reservoirs auf, die voneinander durch zumindest eine Trennwand getrennt sind. Mit anderen Worten stehen diese Reservoirs nicht in Fluidkommunikation miteinander. Dies hat den Vorteil, dass mehrere Punktionsvorgänge (mit darauf folgenden Bestrahlungen) unabhängig voneinander ermöglicht werden. Beispielsweise kann ein erstes Reservoir mit der Photosensibilisator-Lösung bei Bedarf unmittelbar nach der Operation geöffnet werden, während ein zweites Reservoir mit der Photosensibilisator-Lösung auch noch Monate später, falls die Infektion (oder eine andere Infektion) wieder aufflammen sollte, zur Verfügung steht.

Es ist für die Stabilität des Implantats förderlich, wenn nur eng definierte Bereiche der Umhüllung mit der Membran ausgestattet sind. Daher ist in einer weiteren bevorzugten Ausführung des erfindungsgemäßen Implantats die Membran durch ein die Membran zumindest teilweise umschließendes Befestigungselement mit einem weiteren Teil der Umhüllung verbunden. Dieses Befestigungselement ist bevorzugt ringförmig.

Zweckmäßigerweise ist das Befestigungselement oder die Membran mit einem Kontrastmittel markiert, um die Auffindbarkeit der Membran für die Punktion im Inneren des Körpers des Patienten zu erleichtern. Unter Kontrastmittel wird hierin jedes Material verstanden, dass die Auffindbarkeit des Befestigungselements bzw. der Membran in einem am lebenden Patienten durchführbaren bildgebenden Verfahren erleichtert, indem es einen Kontrast in diesem Verfahren liefert. Das Kontrastmittel kann fest oder flüssig sein, bevorzugt ist es fest, insbesondere ist es ein röntgendichtes Material.

Damit das Licht zur Anregung des Photosensibilisators während der Bestrahlung mit stärkerer Intensität auch in Gewebebereiche gelangt, die von der Haut des Patienten weiter entfernt liegen, kann das Implantat Lichtwellenleiter aufweisen. Daher ist in einer weiteren Ausführungsform der vorliegenden Erfindung am bzw. im Implantat zumindest ein Lichtwellenleiter zum Einkoppeln von Licht mit einer Anregungsfrequenz des Photosensibilisators an einer ersten Stelle (z.B. einer Stelle, die in der Nähe zur Haut liegt, wenn das Implantat in den Körper des Patienten eingesetzt worden ist; bei Brustimplantaten z.B. die ventrale Oberfläche des Brustimplantats) und zum Auskoppeln des Lichts an einer zweiten Stelle (z.B. einer Stelle, die weiter entfernt von der Haut liegt, wenn das Implantat in den Körper des Patienten eingesetzt worden ist; bei Brustimplantaten z.B. die dorsale Oberfläche des Brustimplantats) vorgesehen.

Es hat sich als vorteilhaft herausgestellt, wenn die Lösung nach der Punktion der Membran langsam aus dem Reservoir freigesetzt wird. Daher steht in einer weiteren Ausführungsform die Lösung im Reservoir unter einem Druck von höchstens 4 bar, bevorzugt höchstens 2 bar, mehr bevorzugt höchstens 1,5 bar, noch mehr bevorzugt höchstens 1,25 bar, insbesondere höchstens 1,1 bar oder gar höchstens 1,05 bar. Als zweckmäßiger Mindestdruck hat sich beispielsweise ein Druck von 1,01 bar herausgestellt.

Insbesondere in schweren Fällen kann eine Kombination von konventionellen Antibiotikatherapien mit der erfindungsgemäßen photodynamischen Therapie gegen die Infektion sinnvoll sein. Daher enthält die Lösung gemäß einer weiteren Ausführungsform ferner zumindest ein Antibiotikum.

Damit das Implantat nicht versehentlich an einer falschen Stelle punktiert wird, ist es von Vorteil, wenn die Umhüllung im Bereich der Membran eine geringere Wandstärke aufweist als in einem weiteren Bereich der Umhüllung.

Die vorliegende Erfindung ist hervorragend für das Gebiet der Mammaaugmentation geeignet. Folglich ist das Implantat der vorliegenden Erfindung bevorzugt ein Brustimplantat.

Unter dem Begriff "Photosensibilisator" wird hierin jeder Photosensibilisator verstanden, der für den Einsatz als Photosensibilisator im Körper von Säugetieren, insbesondere von Menschen, geeignet ist. Bevorzugt ist der Photosensibilisator ausgewählt aus der Gruppe umfassend Methylenblau, Toluidinblau, Hypericin, Curcumin und Chlorophyll. Methylenblau und Toluidinblau haben sich im Zuge der vorliegenden Erfindung als ganz besonders geeignet in der Bekämpfung von Bakterien der Gattungen *Staphylococcus* (in diesem Fall insbesondere Methylenblau) bzw. *Escherichia* herausgestellt, daher ist es besonders bevorzugt, wenn zumindest einer von diesen Photosensibilisatoren in der Lösung im Reservoir enthalten ist. Weitere geeignete Photosensibilisatoren sind Photosensibilisatoren, die beispielsweise in Dai et al. (wie oben zitiert), der EP 2 186 862 A2 oder der WO 2009/066294 A1 genannt sind.

Unter dem Ausdruck "biokompatibel" wird hierin eine Eignung für einen dauerhaften Einsatz (z.B. zumindest einen Monat, bevorzugt zumindest ein Jahr, insbesondere zumindest fünf Jahre) innerhalb des Säugetierkörpers, bevorzugt des menschlichen Körpers, verstanden. Beispielsweise weist ein biokompatibles Implantat eine möglichst geringe Immunogenität auf.

Die Erfindung wird nachfolgend anhand von besonders bevorzugten Ausführungsbeispielen, auf die sie jedoch nicht eingeschränkt ist, und unter Bezugnahme auf Zeichnungen noch weiter erläutert. Die Zeichnungen zeigen im Einzelnen:
**Fig. 1** zeigt eine teilweise geschnittene perspektivische Darstellung des erfindungsgemäßen Implantats in einer Ausführung als Brustimplantat;
**Fig. 2** zeigt eine schematische Darstellung des als Brustimplantat ausgeführten erfindungsgemäßen Implantats im Körper einer Patientin;
**Fig. 3** zeigt eine seitliche Ansicht des als Brustimplantat ausgeführten erfindungsgemäßen Implantats während der Punktion mit der Nadel;
**Fig. 4** zeigt einen teilweisen Querschnitt des als Brustimplantat ausgeführten erfindungsgemäßen Implantats;
**Fig. 5** zeigt eine Ausführung des als Brustimplantat ausgeführten erfindungsgemäßen Implantats in der Draufsicht;
**Fig. 6** zeigt eine weitere Ausführung des erfindungsgemäßen Implantats als Brustimplantat mit Lichtwellenleitern im Querschnitt; und
**Fig. 7** zeigt eine schematische Darstellung einer Ausführung des erfindungsgemäßen Implantats zur Modellierung der Bauchmuskulatur im Körper eines Patienten.

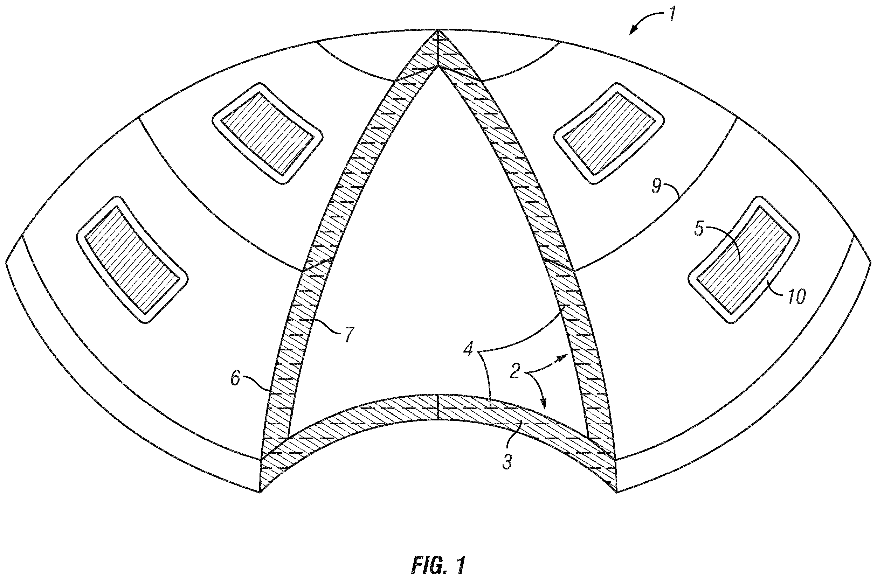

Figur 1 zeigt das als Brustimplantat ausgeführte biokompatible Implantat 1 in teilweise geschnittener Ansicht. Die jeweils von einer Umhüllung 2 definierten Reservoirs 3 enthalten die wässrige Lösung 4 und sind durch Trennwände 9 voneinander getrennt. Die Lösung 4 ist phosphatgepuffert bei physiologischem pH und enthält beispielsweise den Photosensibilisator Methylenblau in einer Konzentration von 1 g/Liter. Jede der Umhüllungen 2 bildet einen Teil der Außenwand 6 und der Innenwand 7, und weist mehrere Membranen 5 auf, wobei diese eine geringere Wandstärke aufweisen als der restliche Teil der Umhüllungen. Jede der Membranen 5 ist gegenüber dem jeweils restlichen Teil der Umhüllung 2 von einem Befestigungselement 10 eingefasst. Die Befestigungselemente 10 enthalten ein röntgendichtes Material, so dass die Membranen 5 mithilfe eines auf Röntgenstrahlen basierenden bildgebenden Verfahrens vom Chirurgen zur Punktion mit der Nadel leicht aufgefunden werden können.

Das Implantat 1 wird der Patientin nach herkömmlichem chirurgischen Verfahren eingesetzt. Bei Diagnose einer mit dem Implantat in Zusammenhang stehenden Infektion punktiert der Chirurg die Membranen 5 mit einer Punktionsnadel, so dass die Lösung 4 aus den Reservoirs 3 ins umliegende infizierte Gewebe austritt. Nach 4 Stunden wird die Patientin mit Licht der Wellenlänge von 632 nm mittels des kommerziell erhältlichen Geräts REPULS^{®} 7 (REPULS Lichtmedizintechnik GmbH, Wien, Österreich) für 10 Minuten bestrahlt. Diese Bestrahlung wird jeweils im Abstand von einigen Stunden wiederholt und der Gesundheitszustand der Patientin wird während dieser Zeit überwacht.

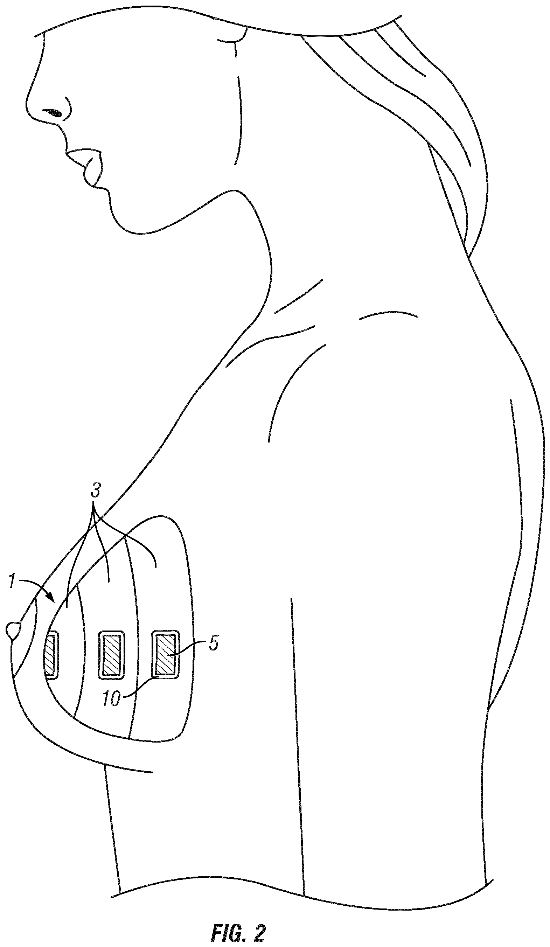

Figur 2 zeigt schematisch das als Brustimplantat ausgebildete Implantat 1 im Körper der Patientin. Das gezeigte Implantat weist drei voneinander unabhängige Reservoirs 3 auf, die miteinander nicht in Fluidkommunikation stehen. Jedes dieser Reservoirs 3 weist zumindest eine punktierbare Membran 5 auf, die von einem röntgendichten Befestigungselement 10 eingefasst ist.

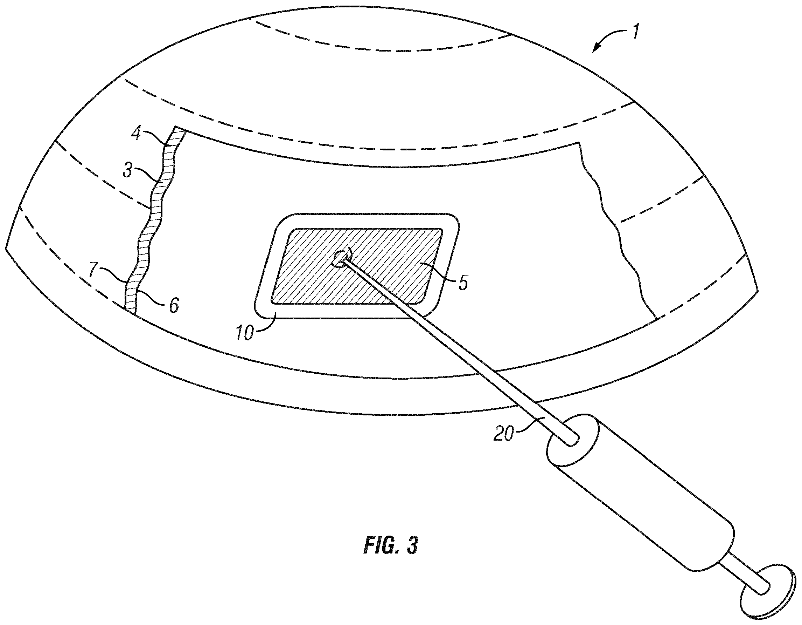

Figur 3 zeigt das als Brustimplantat ausgebildete Implantat 1, während es von der Nadel 20 punktiert wird. Das Reservoir 3 mit der pharmazeutisch akzeptablen Lösung 4, die den Photosensibilisator Toluidinblau enthält, liegt zwischen der Außenwand 6 des Implantats 1 und der Innenwand 7 des Implantats 1.

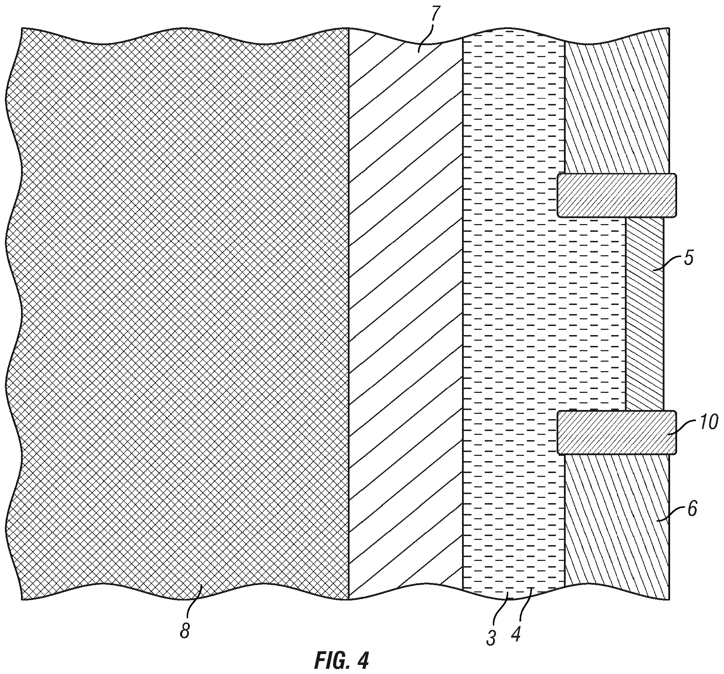

Figur 4 zeigt einen teilweisen Querschnitt des als Brustimplantat ausgebildeten Implantats 1. Die Außenwand 6 des Implantats 1 bzw. die Innenwand 7 des Implantats 1 weist eine höhere Wandstärke als die Membran 5 auf, die durch das Befestigungselement 10 mit der Außenwand 6 verbunden ist. Die Innenwand 7 umgibt die Implantatfüllung 8, welche aus Silikongel besteht, und steht in direktem Kontakt mit der Implantatfüllung 8. Zwischen der Außenwand 6 und der Innenwand 7 liegt das Reservoir 3, das die Lösung 4 mit dem Photosensibilisator enthält.

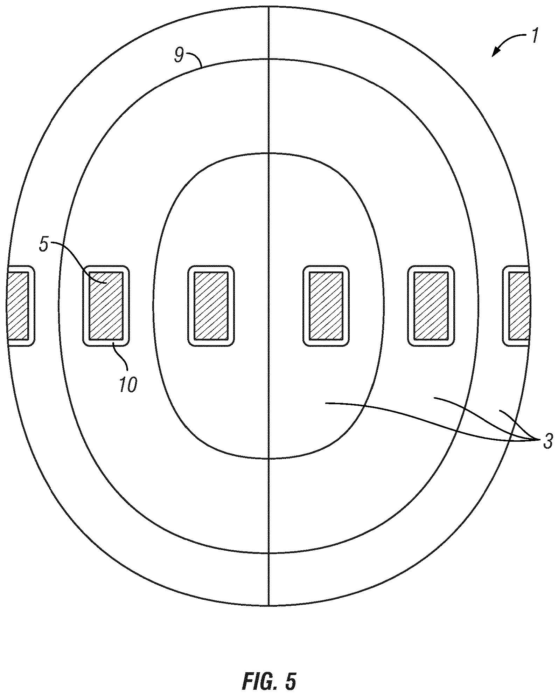

Figur 5 zeigt das als Brustimplantat ausgebildete Implantat 1 in der Draufsicht. Die Reservoirs 3 werden von den Trennwänden 9 voneinander getrennt und sind unabhängig voneinander jeweils durch eine von einem Befestigungselement 10 umschlossene Membran 5 durch Punktion mit einer Nadel zugänglich.

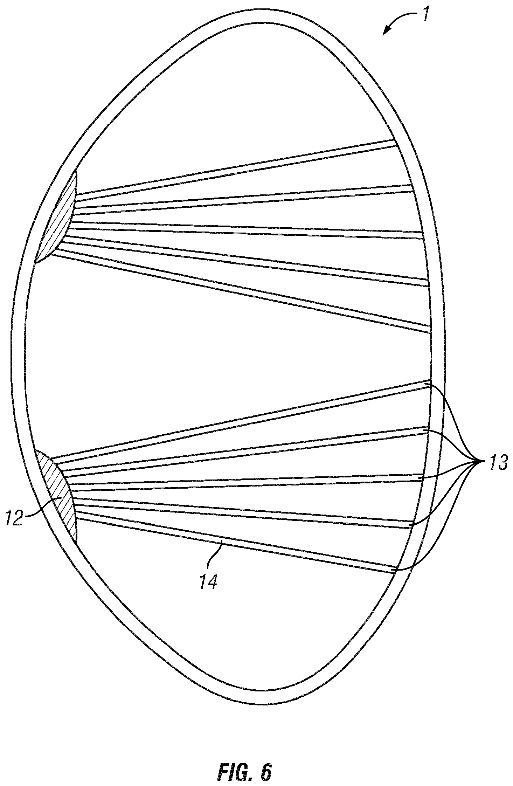

Figur 6 zeigt eine weitere Ausführung des als Brustimplantat ausgebildeten Implantats 1 mit Lichtwellenleitern 14 im Querschnitt. Bei Bestrahlung wird das Licht an einer ersten Stelle 12 in die Lichtwellenleiter 14 eingekoppelt und an zumindest einer zweiten Stelle 13 aus den Lichtwellenleitern 14 ausgekoppelt. Die erste Stelle 12 ist nach Implantation des Implantats 1 ventral gelegen, während die zweite Stelle 13 dorsal gelegen ist. Auf diese Weise kann auch umliegendes Gewebe besser bestrahlt werden, das weiter entfernt von der Oberfläche des Körpers des Patienten ist.

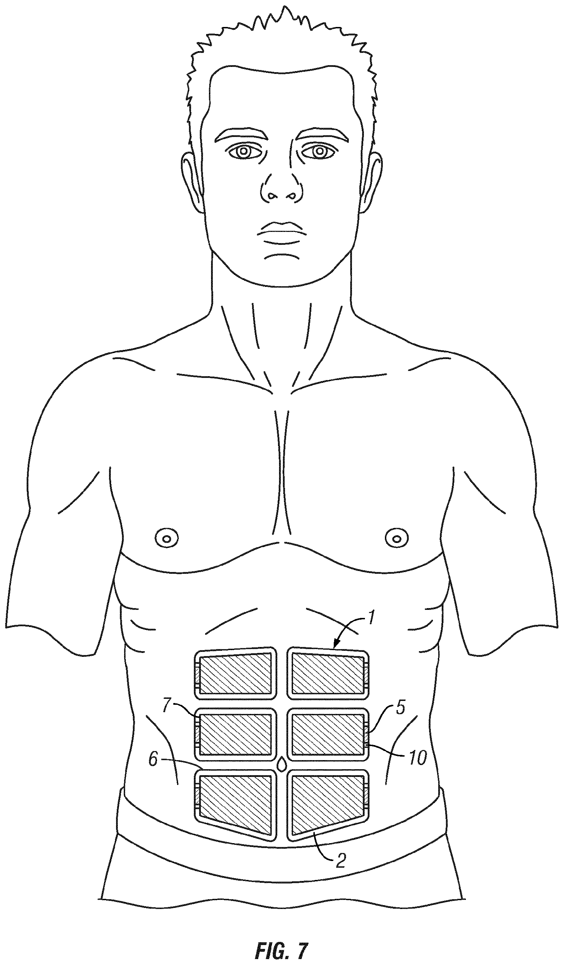

Figur 7 zeigt eine schematische Darstellung einer Ausführung des erfindungsgemäßen Implantats 1 zur Modellierung der Bauchmuskulatur im Körper eines Patienten. Mehrere der Implantate 1, die jeweils eine Implantatfüllung 8 aus Silikongel aufweisen, wurden in den Körper des Patienten eingesetzt. Jedes dieser Implantate 1 weist eine ein Reservoir 3 mit Photosensibilisator-Lösung umgebende Umhüllung 2 auf, die durch eine punktierbare Membran 5, die Teil der Außenwand 6 ist, zugänglich ist.

## Patentansprüche

1. Biokompatibles Implantat (1) für einen Patienten, wobei das Implantat (1) zumindest ein von einer Umhüllung (2) definiertes Reservoir (3), das eine pharmazeutisch annehmbare Lösung (4) enthält, aufweist, **dadurch gekennzeichnet, dass** die Lösung (4) zumindest einen Photosensibilisator enthält, und dass zumindest ein Teil der Umhüllung (2) des zumindest einen Reservoirs (3) durch eine Membran (5) gebildet ist, wobei die Membran so konfiguriert ist, dass sie von einer Nadel (20) punktierbar ist, so dass durch Punktion mit der Nadel (20) zumindest ein Teil der Lösung (4) aus dem Reservoir (3) in die Umgebung des Implantats (1) freisetzbar ist.

2. Biokompatibles Implantat (1) gemäß Anspruch 1, **dadurch gekennzeichnet, dass** ein erster Teil der Umhüllung (2), welcher die Membran (5) aufweist, eine Außenwand (6) des Implantats (1) bildet und ein zweiter Teil der Umhüllung (2) eine Innenwand (7) des Implantats (1) bildet, wobei die Innenwand (7) zumindest teilweise eine Implantatfüllung (8) umgibt.

3. Biokompatibles Implantat (1) gemäß Anspruch 1 oder 2, **dadurch gekennzeichnet, dass** das Implantat (1) eine Vielzahl an Reservoirs (3) aufweist, die voneinander durch zumindest eine Trennwand (9) getrennt sind.

4. Biokompatibles Implantat (1) gemäß einem der Ansprüche 1 bis 3, **dadurch gekennzeichnet, dass** die Membran (5) durch ein die Membran (5) zumindest teilweise umschließendes Befestigungselement (10) mit einem weiteren Teil der Umhüllung (2) verbunden ist.

5. Biokompatibles Implantat (1) gemäß Anspruch 4, **dadurch gekennzeichnet, dass** das Befestigungselement (10) oder die Membran (5) mit einem Kontrastmittel, bevorzugt einem röntgendichten Material, markiert ist.

6. Biokompatibles Implantat (1) gemäß einem der Ansprüche 1 bis 5, **dadurch gekennzeichnet, dass** zumindest ein Lichtwellenleiter (14) zum Einkoppeln von Licht mit einer Anregungsfrequenz des Photosensibilisators an einer ersten Stelle (12) und zum Auskoppeln des Lichts an einer zweiten Stelle (13) vorgesehen ist.

7. Biokompatibles Implantat (1) gemäß einem der Ansprüche 1 bis 6, **dadurch gekennzeichnet, dass** die Lösung (4) im Reservoir (3) unter einem Druck von höchstens 4 bar, bevorzugt höchstens 2 bar, mehr bevorzugt höchstens 1,5 bar, noch mehr bevorzugt höchstens 1,25 bar, insbesondere höchstens 1,1 bar oder gar höchstens 1,05 bar steht.

8. Biokompatibles Implantat (1) gemäß einem der Ansprüche 1 bis 7, **dadurch gekennzeichnet, dass** die Lösung (4) ferner zumindest ein Antibiotikum enthält.

9. Biokompatibles Implantat (1) gemäß einem der Ansprüche 1 bis 8, **dadurch gekennzeichnet, dass** die Umhüllung (2) im Bereich der Membran (5) eine geringere Wandstärke aufweist als in einem weiteren Bereich der Umhüllung (2).

10. Biokompatibles Implantat (1) gemäß einem der Ansprüche 1 bis 9, **dadurch gekennzeichnet, dass** es ein Brustimplantat ist.

11. Biokompatibles Implantat (1) gemäß einem der Ansprüche 1 bis 10, **dadurch gekennzeichnet, dass** der Photosensibilisator Methylenblau ist.

12. Biokompatibles Implantat (1) gemäß einem der Ansprüche 1 bis 10, **dadurch gekennzeichnet, dass** der Photosensibilisator Toluidinblau ist.

## Claims

1. Biocompatible implant (1) for a patient, the implant (1) having at least one reservoir (3) which is defined by a casing (2) and contains a pharmaceutically acceptable solution (4), **characterised in that** the solution (4) contains at least one photosensitiser, and **in that** at least part of the casing (2) of the at least one reservoir (3) is formed by a membrane (5), the membrane being configured such that it can be punctured by a needle (20), such that at least some of the solution (4) can be released from the reservoir (3) into the surroundings of the implant (1) by puncturing with the needle (20).

2. Biocompatible implant (1) according to claim 1, **characterised in that** a first part of the casing (2), which has the membrane (5), forms an outer wall (6) of the implant (1) and a second part of the casing (2) forms an inner wall (7) of the implant (1), the inner wall (7) at least partially surrounding an implant filling (8).

3. Biocompatible implant (1) according to either claim 1 or claim 2, **characterised in that** the implant (1) has a plurality of reservoirs (3) which are separated from one another by at least one partition (9).

4. Biocompatible implant (1) according to any of claims 1 to 3, **characterised in that** the membrane (5) is connected to a further part of the casing (2) by a fastening element (10) which at least partially encloses the membrane (5).

5. Biocompatible implant (1) according to claim 4, **characterised in that** the fastening element (10) or the membrane (5) is marked with a contrast agent, preferably a radiopaque material.

6. Biocompatible implant (1) according to any of claims 1 to 5, **characterised in that** at least one optical waveguide (14) in provided for coupling in light with an excitation frequency of the photosensitiser at a first point (12) and for coupling out the light at a second point (13).

7. Biocompatible implant (1) according to any of claims 1 to 6, **characterised in that** the solution (4) in the reservoir (3) is under a pressure of at most 4 bar, preferably at most 2 bar, more preferably at most 1.5 bar, even more preferably at most 1.25 bar, in particular at most 1.1 bar or even at most 1.05 bar.

8. Biocompatible implant (1) according to any of claims 1 to 7, **characterised in that** the solution (4) also contains at least one antibiotic.

9. Biocompatible implant (1) according to any of claims 1 to 8, **characterised in that** the casing (2) has a smaller wall thickness in the region of the membrane (5) than in a further region of the casing (2).

10. Biocompatible implant (1) according to any of claims 1 to 9, **characterised in that** it is a breast implant.

11. Biocompatible implant (1) according to any of claims 1 to 10, **characterised in that** the photosensitiser is methylene blue.

12. Biocompatible implant (1) according to any of claims 1 to 10, **characterised in that** the photosensitiser is toluidine blue.

## Revendications

1. Implant biocompatible (1) pour un patient, où l'implant (1) comporte au moins un réservoir (3) défini par une enveloppe (2), qui contient une solution pharmaceutiquement acceptable (4), **caractérisé en ce que** la solution (4) contient au moins un photosensibilisateur, et **en ce qu'**au moins une partie de l'enveloppe (2) du au moins un réservoir (3) est formée par une membrane (5), où la membrane est configurée de telle manière qu'elle peut être perforée par une aiguille (20) de sorte qu'au moins une partie de la solution (4) peut être libérée du réservoir (3) dans l'environnement de l'implant (1) par perforation avec l'aiguille (20).

2. Implant biocompatible (1) selon la revendication 1, **caractérisé en ce qu'**une première partie de l'enveloppe (2), qui comporte la membrane (5), forme une paroi externe (6) de l'implant (1) et une deuxième partie de l'enveloppe (2) forme une paroi interne (7) de l'implant (1), où la paroi interne (7) entoure au moins partiellement un garnissage d'implant (8).

3. Implant biocompatible (1) selon la revendication 1 ou 2, **caractérisé en ce que** l'implant (1) comporte une pluralité de réservoirs (3) qui sont séparés les uns des autres par au moins une paroi de séparation (9).

4. Implant biocompatible (1) selon l'une des revendications 1 à 3, **caractérisé en ce que** la membrane (5) est reliée à une autre partie de l'enveloppe (2) par un élément de fixation (10) qui entoure au moins partiellement la membrane (5).

5. Implant biocompatible (1) selon la revendication 4, **caractérisé en ce que** l'élément de fixation (10) ou la membrane (5) est marqué avec un agent de contraste, de préférence un matériau radio-opaque.

6. Implant biocompatible (1) selon l'une des revendications 1 à 5, **caractérisé en ce qu'**au moins un guide d'onde optique (14) pour l'injection de lumière ayant une fréquence d'excitation du photosensibilisateur en un premier point (12) et pour la sortie de la lumière en un deuxième point (13) est prévu.

7. Implant biocompatible (1) selon l'une des revendications 1 à 6, **caractérisé en ce que** la solution (4) dans le réservoir (3) est sous une pression d'au plus 4 bar, de préférence d'au plus 2 bar, de préférence encore d'au plus 1,5 bar, de préférence encore d'au plus 1,25 bar, en particulier d'au plus 1,1 bar ou même d'au plus 1,05 bar.

8. Implant biocompatible (1) selon l'une des revendications 1 à 7, **caractérisé en ce que** la solution (4) contient en outre au moins un antibiotique.

9. Implant biocompatible (1) selon l'une des revendications 1 à 8, **caractérisé en ce que** l'enveloppe (2) présente dans le domaine de la membrane (5) une épaisseur de paroi plus faible que dans un autre domaine de l'enveloppe (2).

10. Implant biocompatible (1) selon l'une des revendications 1 à 9, **caractérisé en ce qu'**il s'agit d'un implant mammaire.

11. Implant biocompatible (1) selon l'une des revendications 1 à 10, **caractérisé en ce que** le photosensibilisateur est le bleu de méthylène.

12. Implant biocompatible (1) selon l'une des revendications 1 à 10, **caractérisé en ce que** le photosensibilisateur est le bleu de toluidine.
